# EUROPEAN PATENT APPLICATION

(11) **EP 3 079 156 A1**
(43) Date of publication of application: **12.10.2016**
(21) Application number: 15162885.6
(22) Date of filing: 09.04.2015
(51) Int. Cl.: H01B 3/56, H01B 3/24

(54) **METHODS FOR DIELECTRICALLY INSULATING ELECTRICAL ACTIVE PARTS**

(71) Applicant: Solvay SA, 1120 Bruxelles (BE)
(72) Inventor: The designation of the inventor has not yet been filed
(74) Representative: Mross, Stefan P.M.

(57) **Abstract**

The invention concerns methods for dielectrically insulating electrical active parts using certain fluorinated dicarbonyl compounds as well as compositions and apparatus comprising such compounds.

## Description

The invention concerns methods for dielectrically insulating electrical active parts using certain fluorinated dicarbonyl compounds, preferably diketones, as well as compositions and apparatus comprising such compounds.

Dielectrically insulation media in liquid or gaseous state are applied for the insulation of electrical active parts in a wide variety of electrical apparatus, e.g. in switchgears or transformers.

Mixtures of SF₆ and N₂ are widely applied as dielectrically insulating medium. Efforts have been made in the past to provide alternative dielectrically insulating media.

WO 2014/096414 concerns a method of dielectrically insulating electrical active parts using certain fluorinated compounds, e.g. fluorinated peroxides.

The object of the present invention is to provide improved methods and/or compositions for the electrical insulation of electrical active parts.

Advantageously, the methods and compositions of the present invention show improved insulation, arc-extinguishing and/or switching performance. Also advantageously, the methods and compositions of the present invention show advantageous environmental impact when the insulating medium is released into the atmosphere, e.g. as measured by an improved global warming potential (GWP) and/or improved ozone depletion. Also advantageously, the methods and compositions of the present invention show an improved toxicological behavior, as measured for example by a higher LC50 and/or a higher Occupational Exposure Limit. Furthermore, the methods and compositions advantageously show an improved dew point, vapour pressure, boiling point, dielectrical strengths, and/or thermal stability of the insulating media. Additionally, the compositions according to this invention advantageously show an improved chemical inertness against the construction materials used e.g. for the electrical active parts and/or improved heat transfer properties.

These and other objectives are solved by the present invention as outlined in the claims.

Accordingly, a first aspect of the present invention concerns a method for dielectrically insulating an electrical active part wherein the electrical active part is arranged in a gas-tight housing comprising an insulating medium consisting of, consisting essentially of, or comprising a compound of general formula (I) R¹-(O)ₙ-C(O)-Z-C(O)-(O)ₘ-R² wherein Z is a single bond, O, NH, NR³, an alkylidene group, a partially fluorinated alkylidene group or a perfluorinated alkylidene group; and R¹, R² and R³ are independently a partially fluorinated or perfluorinated alkyl group; or R¹ and R² together form a partially fluorinated or perfluorinated alkylidene group and R³ is a partially fluorinated or perfluorinated alkyl group; and n and m are independently 0 or 1.

When Z is a single bond, the two carbonyl groups are adjacent to each other and the compound comprises a moiety of the structure -C(O)-C(O)-, i.e. an α,β-diketone.

The term "consisting essentially of" as used herein is intended to denote a composition comprising the components as specified as well as other components in trace amounts wherein the presence of the other components does not change the essential characteristics of the specified subject matter.

Preferably, the compounds used in the method independently comprise from 1 to 5 carbon atoms in residues R¹ and R².

Preferably, R¹ and R² are perfluorinated, i.e. R¹ and R² are CₙF₂ₙ₊₁. Suitable examples include perfluorinated methyl, ethyl, isopropyl, n-propyl, isobutyl, n-butyl or tert-butyl, n-pentyl or isopentyl groups, preferably trifluoromethyl, pentafluoroethyl, and heptafluoroisopropyl, most preferably R¹ and R² are CF₃. R³ is most preferably C₂F₅ or CF₃, specifically CF₃.

Alternatively, R¹ and R² are not perfluorinated. In this case, R¹ and R² are independently chosen from the group consisting of partially fluorinated methyl, ethyl, isopropyl, n-propyl, isobutyl, n-butyl, tert-butyl, n-pentyl and isopentyl. In theses alternative embodiment, R¹ and R² are preferably chosen from difluoromethyl, tetrafluoroethyl, n-hexafluoropropyl and isohexafluoropropyl, more preferably difluoromethyl.

R¹ and R² can preferably be the same. In an alternative preferred embodiment, R¹ and R² are different.

Also preferably, n and m are 0.

Also preferably, Z is a single bond or a perfluorinated alkylidene group, more preferably Z is CF₂.

Also preferred are embodiments wherein n and m are 0 and Z is a single bond or a perfluorinated alkylidene group, more preferably Z is CF₂. Thus, in this especially preferred embodiments the insulating medium consists of, consists essentially of, or comprises diketones, specifically the insulating medium consists of or consists essentially of diketones and at least one inert gas.

In most preferred embodiments of this aspect, the compound of general formula (I) is CF₃C(O)CF₂C(O)CF₃, CF₃C(O)C(O)CF₃, CF₃CF₂C(O)CF₂C(O)CF₂CF₃, or CF₃CF₂C(O) C(O)CF₂CF₃, specifically CF₃C(O)CF₂C(O)CF₃.

In the frame of the present invention, the singular is intended to include the plural, and vice versa.

Compounds of general formula (I) can be obtained commercially or prepared by methods known in the prior art. For example, Conti, S. et al., Acta Chimica Slovenica, 2013, 60(3), 556-560 discloses the synthesis of CF₃C(O)CF₂C(O)CF₃.

The anhydride compounds (Z=O) and the imide compounds (Z=NH, NR³) can for example be prepared from the corresponding partially or perfluorinated carboxylic acids or carboxylic acid chlorides by methods known to the skilled person, for example following a reaction

2 CF₃C(O)Cl + NH₃ → CF₃C(O)NHC(O)CF₃.

The α,β-diketones (Z is a single bond) can for example be prepared from the corresponding carboxylic acid chlorides via a Corey-Seebach reaction:

2 CF₃C(O)Cl → CF₃C(O)C(O)CF₃.

Preferably, the insulating medium used in the inventive method comprises the compound of formula (I) and at least one further compound selected from the list consisting of an inert gas, a perfluorinated or partially fluorinated monoketone, a perfluorinated or partially fluorinated ether, a perfluorinated or partially fluorinated cyano compound and a hydrocarbon compound. More preferably, the at least one compound is an inert gas selected from the group consisting of air, synthetic air, an air component, N₂, O₂, CO₂, N₂O, He, Ne, Ar, Xe and SF₆; preferably the at least one compound is N₂.

The term "inert gas" is intended to denote a gas that does not react with the compounds according to the invention. Preferably, the inert gas is chosen from the list consisting of air, synthetic air, an air component, N₂, O₂, CO₂, N₂O, He, Ne, Ar, Xe or SF₆; more preferably, the inert gas is N₂.

Preferably, the at least one compound is a perfluorinated or partially fluorinated monoketone. The term "monoketone" is intended to denote a compound incorporating one carbonyl group with two carbon atoms attached to the carbon of the carbonyl group. It shall encompass saturated compounds and unsaturated compounds including double and/or triple bonds. The at least partially fluorinated alkyl chain of the ketones can be linear or branched. The term "monoketone" shall also encompass compounds with a cyclic carbon backbone. More preferably, the at least one compound is a perfluorinated ketone. Examples of suitable perfluorinated ketones include 1,1,1,3,4,4,4-heptafluoro-3-(trifluoromethyl)-butan-2-one; 1,1,1,3,3,4,4,5,5,5-decafluoropentan-2-one; 1,1,1,2,2,4,4,5,5,5-decafluoropentan-3-one, 1,1,1,4,4,5,5,5,-octafluoro-3-bis-(trifluoromethyl)-pentan-2-one; and most preferably heptafluoroisopropyl-trifluoromethylketone.

Also preferably, the at least one compound is a perfluorinated or partially fluorinated ether. The term "ether" is intended to denote a compound incorporating at least one "-C-O-C-" moiety. Especially suitable examples include pentafluoro-ethyl-methyl ether and 2,2,2-trifluoroethyl-trifluoromethyl ether.

Also preferably, the at least one compound is a perfluorinated or partially fluorinated cyano compound, i.e. a compound incorporating at least one moiety of the structure "-C=N". Preferably, the cyano compound is perfluorinated, more preferably the cyano compound is chosen from the list consisting of perfluorinated methyl, ethyl, isopropyl, propyl, butyl, isobutyl and tertbutyl nitrile.

Also preferably, the at least one compound is a perfluorinated or partially fluorinated hydrocarbon compound. "Hydrocarbon compound" is intended to denote a saturated or unsaturated hydrocarbon, which may in addition to the fluoro substitution also be substituted by other halogen atoms, e.g. Cl, Br, and/or I. Suitable examples include CHF₃, C₂F₄, CF₃CF₂CF₂CF₂I, and CF₂Cl₂.

The term "electrical active part" has to be understood very broadly. Preferably, it covers any part which is used for the generation, the distribution or the usage of electrical energy provided it comprises a gas-tight housing wherein the dielectrically insulating medium provides for the dielectrically insulation of parts which bear voltage or current. Preferably, the electrical active parts are medium voltage or high voltage parts. The term "medium voltage" relates to a voltage in the range of 1 kV to 72 kV; the term "high voltage" refers to a voltage of more than 72 kV. While these are preferred electrical active parts in the frame of the present invention, the parts may also be low voltage parts with a voltage below 1 kV being concerned.

It has to be noted that the electrical active parts of the invention can be "stand alone" parts, or they can be part of an assembly of parts, e.g. of an apparatus. This will now be explained in detail.

The electrical active part can be a switch, for example, a fast acting earthing switch, a disconnector, a load-break switch or a puffer circuit breaker, in particular a medium-voltage circuit breaker (GIS-MV), a generator circuit breaker (GIS-HV), a high voltage circuit breaker, a bus bar a bushing, a gas-insulated cable, a gas-insulated transmission line, a cable joint, a current transformer, a voltage transformer or a surge arrester.

The electrical active part may also be part of an electrical rotating machine, a generator, a motor, a drive, a semiconducting device, a computing machine, a power electronics device or high frequency parts, for example, antennas or ignition coils.

The method of the invention is especially suited for medium voltage switchgears and high voltage switchgears.

In the electrical active part, the insulating medium is preferably at a pressure of equal to or greater than 0.1 bar (abs.). The insulating medium is preferably at a pressure equal to or lowers than 30 bar (abs). A preferred pressure range is from 1 to 20 bar (abs.).

The partial pressure of the compound of general structure (I) in the gaseous phase depends, i.a. upon its concentration in the isolating medium. If the dielectrically isolating medium consists of the compound of general structure (I) its partial pressure is equal to the total pressure and corresponds to the ranges given above. If the medium includes an inert gas, the partial pressure of the compound of general structure (I) is correspondingly lower. A partial pressure of the compound of general structure (I) which is equal to or lower than 10 bar (abs) is preferred.

It is also preferred that the compound or the composition, respectively, is such that under the climate conditions or the temperature in the ambience of the electrical apparatus, under the pressure in the electrical part, essentially no condensation of the components in the dielectrically insulating medium occurs. The term "essentially no condensation" denotes that at most 5 % by weight, preferably at most 2 % by weight, of the dielectrically insulating medium condenses. For example, the amounts of compound of formula (I) the kind and amount of inert gas are selected such that the partial pressure of compound of formula (I) is lower than the pressure where condensation of compound of formula (I) is observed at -20°C. However, depending on the conditions, e.g. the temperature and the pressure, under which the method is performed, the insulating medium can also be, at least partially, in the liquid state.

In a second aspect, the present invention concerns a composition consisting of, consisting essentially of, or comprising at least one compound of general formula (I): R¹-(O)ₙ-C(O)-Z-C(O)-(O)ₘ-R² wherein Z is a single bond, O, NH, NR₃, an alkylidene group, a partially fluorinated alkylidene group or a perfluorinated alkylidene group; and R¹, R² and R³ are independently a partially fluorinated or perfluorinated alkyl group; or R¹ and R² together form a partially fluorinated or perfluorinated alkylidene group and R³ is a partially fluorinated or perfluorinated alkyl group; and n and m are independently 0 or 1; and at least one further compound selected from the group consisting of an inert gas, a perfluorinated or partially fluorinated ketone, a perfluorinated or partially fluorinated ether, a perfluorinated or partially fluorinated ester, a perfluorinated or partially fluorinated cyano compound and a hydrocarbon compound.

Preferably, the composition consists of, consists essentially of, or comprises CF₃C(O)CF₂C(O)CF₃, CF₃C(O)C(O)CF₃, CF₃CF₂C(O)CF₂C(O)CF₂CF₃, or CF₃CF₂C(O) C(O)CF₂CF₃, preferably CF₃C(O)CF₂C(O)CF₃, and at least one compound selected from the group consisting of an inert gas, a perfluorinated or partially fluorinated ketone, a perfluorinated or partially fluorinated ether, a perfluorinated or partially fluorinated ester, a perfluorinated or partially fluorinated cyano compound and a hydrocarbon compound.

Also preferably, the composition consists of, consists essentially of, or comprises CF₃C(O)CF₂C(O)CF₃ and at least one compound selected from the group consisting of air, synthetic air, an air component, N₂, O₂, CO₂, N₂O, He, Ne, Ar, Xe or SF₆.

In a third aspect, the present invention concerns an apparatus for the generation, distribution and/or usage of electrical energy wherein the apparatus comprises an electrical active part arranged in a gas-tight housing and said gas-tight housing containing an insulating medium comprising, consisting essentially of, or consisting of at least one compound of general formula (I) as described above or a composition according to this invention as described above. Preferably, the insulating medium consists of, consists essentially of, or comprises CF₃C(O)CF₂C(O)CF₃, CF₃C(O)C(O)CF₃, CF₃CF₂C(O)CF₂C(O)CF₂CF₃, or CF₃CF₂C(O) C(O)CF₂CF₃, specifically CF₃C(O)CF₂C(O)CF₃. Also preferably, the apparatus is a switchgear, more preferably a switchgear for medium or high voltage.

Another aspect of the present invention concerns the use of the compounds of general formula (I) or the compositions of this invention as dielectrically insulating medium or as constituent of a dielectrically insulating medium as well as their use as an dry etching agent, e.g. a chamber cleaning agent, specifically, for plasma-enhanced chamber cleaning as a replacement for NF₃.

Should the disclosure of any patents, patent applications, and publications which are incorporated herein by reference conflict with the description of the present application to the extent that it may render a term unclear, the present description shall take precedence.

The following examples further explain the invention without intention to limit it.

### Examples

### Example 1a: Manufacture of CF3C(O)CF2C(O)CF3

CF₃C(O)CF₂C(O)CF₃ was prepared according to Conti, S. et al., Acta Chimica Slovenica, 2013, 60(3), 556-560.

### Example 1b: Manufacture of the compositions

As described in WO98/23363, a homogenous mixture consisting CF₃C(O)CF₂C(O)CF₃ and N₂ in a volume ratio 1:4 is manufactured in an apparatus comprising a static mixer and a compressor.

### Example 2: Provision of an earth cable containing the dielectrically insulting medium of example 1

The composition of example 1b is directly fed into an earth cable for high voltage, until a total pressure of 10 bar (abs) is achieved in the cable.

### Example 3: A switchgear containing CF₃C(O)CF₂C(O)CF₃ and N₂ in a volume ratio 1:4

A switchgear is used which contains a switch surrounded by a gas-tight metal case. The composition of example b1 is passed into the gas tight metal case via a valve until a pressure of 18 bar (abs) is achieved.

## Claims

1. A method for dielectrically insulating an electrical active part
wherein the electrical active part is arranged in a gas-tight housing comprising an insulating medium consisting of, consisting essentially of, or comprising a compound of general formula (I):
R¹-(O)ₙ-C(O)-Z-C(O)-(O)ₘ-R² (I)
wherein Z is a single bond, O, NH, NR³, an alkylidene group, a partially fluorinated alkylidene group or a perfluorinated alkylidene group;
and R¹, R² and R³ are independently a partially fluorinated or perfluorinated alkyl group;
or R¹ and R² together form a partially fluorinated or perfluorinated alkylidene group and R³ is a partially fluorinated or perfluorinated alkyl group;
and n and m are independently 0 or 1.

2. The method according to claim 1 wherein n and m are 0.

3. The method according to claim 1 or 2 wherein Z is a single bond or a perfluorinated alkylidene group, preferably Z is -CF₂-.

4. The method according to any one of claims 1 to 3 wherein R¹ and R² are independently -CₙF₂ₙ₊₁.

5. The method according to any one of claims 1 to 4 wherein R¹ and R² are -CF₃ or -C₂F₅.

6. The method according to any one of claims 1 to 5 wherein the compound is CF₃C(O)CF₂C(O)CF₃, CF₃C(O)C(O)CF₃, CF₃CF₂C(O)CF₂C(O)CF₂CF₃, or CF₃CF₂C(O) C(O)CF₂CF₃, preferably CF₃C(O)CF₂C(O) CF₃.

7. The method according to any one of claims 1 to 6 wherein the insulating medium comprises the compound of formula (I) and at least one further compound selected from the list consisting of an inert gas, a perfluorinated or partially fluorinated monoketone, a perfluorinated or partially fluorinated ether, a perfluorinated or partially fluorinated cyano compound and a hydrocarbon compound.

8. The method according to claim 7 wherein the at least one compound is an inert gas selected from the group consisting of air, synthetic air, an air component, N₂, O₂, CO₂, N₂O, He, Ne, Ar, Xe and SF₆; preferably the at least one compound is N₂.

9. A composition consisting of, consisting essentially of, or comprising at least one compound of general formula (I)
R¹-Oₙ-C(O)-Z-C(O)-Oₘ-R² (I)
wherein Z is a single bond, O, NH, NR³, an alkylidene group, a partially fluorinated alkylidene group or a perfluorinated alkylidene group;
and R¹, R² and R³ are independently a partially fluorinated or perfluorinated alkyl group;
or R¹ and R² together form a partially fluorinated or perfluorinated alkylidene group and R³ is a partially fluorinated or perfluorinated alkyl group;
and n and m are independently 0 or 1;
and at least one further compound selected from the group consisting of an inert gas, a perfluorinated or partially fluorinated monoketone, a perfluorinated or partially fluorinated ether, a perfluorinated or partially fluorinated cyano compound and a hydrocarbon compound.

10. The composition according to claim 9 consisting of, consisting essentially of, or comprising CF₃C(O)CF₂C(O)CF₃, CF₃C(O)C(O)CF₃, CF₃CF₂C(O)CF₂C(O)CF₂CF₃, or CF₃CF₂C(O) C(O)CF₂CF₃, preferably CF₃C(O)CF₂C(O)CF₃, and at least one compound selected from the group consisting of an inert gas, a perfluorinated or partially fluorinated monoketone, a perfluorinated or partially fluorinated ether, a perfluorinated or partially fluorinated cyano compound and a hydrocarbon compound.

11. The composition of claim 9 or 10 consisting of, consisting essentially of, or comprising CF₃C(O)CF₂C(O)CF₃ and at least one compound selected from the group consisting of air, synthetic air, an air component, N₂, O₂, CO₂, N₂O, He, Ne, Ar, Xe or SF₆; preferably consisting of, consisting essentially of, or comprising O=P(CF₃)₃ and N₂.

12. An apparatus for the generation, distribution and/or usage of electrical energy wherein the apparatus comprises an electrical active part arranged in a gas-tight housing, said gas-tight housing containing an insulating medium consisting of, consisting essentially of, or comprising at least one compound of general formula (I)
R¹-(O)ₙ-C(O)-Z-C(O)-(O)ₘ-R² (I)
wherein Z is a single bond, O, NH, NR³, an alkylidene group, a partially fluorinated alkylidene group or a perfluorinated alkylidene group;
and R¹, R² and R³ are independently a partially fluorinated or perfluorinated alkyl group;
or R¹ and R² together form a partially fluorinated or perfluorinated alkylidene group and R³ is a partially fluorinated or perfluorinated alkyl group;
and n and m are independently 0 or 1;
or containing an insulating medium consisting of, consisting essentially of, or comprising the composition according to any one of claims 9 to 11.

13. The apparatus of claim 12 wherein the insulating medium consists of, consists essentially of, or comprises CF₃C(O)CF₂C(O)CF₃, CF₃C(O)C(O)CF₃, CF₃CF₂C(O)CF₂C(O)CF₂CF₃, or CF₃CF₂C(O) C(O)CF₂CF₃, preferably CF₃C(O)CF₂C(O)CF₃.

14. The apparatus of claim 12 or 13 wherein the apparatus is a switchgear.
